# EUROPEAN PATENT APPLICATION

(11) **EP 2 495 317 A1**
(43) Date of publication of application: **05.09.2012**
(21) Application number: 11156367.2
(22) Date of filing: 01.03.2011
(51) Int. Cl.: C12N 9/88, C12P 13/04

(54) **Modified phosphoenolpyruvate carboxylase from Corynebacterium glutamicum and uses thereof**

(71) Applicant: Technische Universität Hamburg-Harburg, 21073 Hamburg (DE); TuTech Innovation GmbH, 21079 Hamburg (DE)
(72) Inventor: Zeng, An-Ping, 21224, Rosengarten (DE); Zhen, Chen, 21073, Hamburg (DE); Rappert, Sugima, 21073, Hamburg (DE)
(74) Representative: UEXKÜLL & STOLBERG

(57) **Abstract**

The present invention relates to methods and enzymes which are useful for producing oxalacetate-derived amino acids such as L-lysine, L-threonine, L-methionine, L-asparagine, L-isoleucine and L-aspartic acid. In particular, the present invention provides a modified phosphoenolpyruvate carboxylase enzyme with a reduced sensitivity to feedback inhibition by aspartic acid, succinic acid and/or malic acid. The invention also provides methods for producing L-lysine, L-threonine, L-methionine, L-asparagine, L-isoleucine or L-aspartic acid which make use of the modified phosphoenolpyruvate carboxylase enzyme. The invention also relates to the use of the modified enzyme for producing one of the above mentioned amino acids.

SEQUENCE LISTING

<110>  Technische Universität Hamburg-Harburg

<120>  Modified phosphoenolpyruvate carboxylase enzymes and uses thereof
<130>  P 84374

<160>  19
<170>  PatentIn version 3.3

<210>  1
<211>  919
<212>  PRT
<213>  Corynebacterium glutamicum

<400>  1

Met Thr Asp Phe Leu Arg Asp Asp Ile Arg Phe Leu Gly Gln Ile Leu
1               5                   10                  15

Gly Glu Val Ile Ala Glu Gln Glu Gly Gln Glu Val Tyr Glu Leu Val
            20                  25                  30

Glu Gln Ala Arg Leu Thr Ser Phe Asp Ile Ala Lys Gly Asn Ala Glu
        35                  40                  45

Met Asp Ser Leu Val Gln Val Phe Asp Gly Ile Thr Pro Ala Lys Ala
    50                  55                  60

Thr Pro Ile Ala Arg Ala Phe Ser His Phe Ala Leu Leu Ala Asn Leu
65                  70                  75                  80

Ala Glu Asp Leu Tyr Asp Glu Glu Leu Arg Glu Gln Ala Leu Asp Ala
                85                  90                  95

Gly Asp Thr Pro Pro Asp Ser Thr Leu Asp Ala Thr Trp Leu Lys Leu
            100                 105                 110

Asn Glu Gly Asn Val Gly Ala Glu Ala Val Ala Asp Val Leu Arg Asn
        115                 120                 125

Ala Glu Val Ala Pro Val Leu Thr Ala His Pro Thr Glu Thr Arg Arg
    130                 135                 140

Arg Thr Val Phe Asp Ala Gln Lys Trp Ile Thr Thr His Met Arg Glu
145                 150                 155                 160

Arg His Ala Leu Gln Ser Ala Glu Pro Thr Ala Arg Thr Gln Ser Lys
                165                 170                 175

Leu Asp Glu Ile Glu Lys Asn Ile Arg Arg Arg Ile Thr Ile Leu Trp
            180                 185                 190

Gln Thr Ala Leu Ile Arg Val Ala Arg Pro Arg Ile Glu Asp Glu Ile
        195                 200                 205

Glu Val Gly Leu Arg Tyr Tyr Lys Leu Ser Leu Leu Glu Glu Ile Pro
    210                 215                 220

Arg Ile Asn Arg Asp Val Ala Val Glu Leu Arg Glu Arg Phe Gly Glu
225                 230                 235                 240

Gly Val Pro Leu Lys Pro Val Val Lys Pro Gly Ser Trp Ile Gly Gly
                245                 250                 255

Asp His Asp Gly Asn Pro Tyr Val Thr Ala Glu Thr Val Glu Tyr Ser
            260                 265                 270

Thr His Arg Ala Ala Glu Thr Val Leu Lys Tyr Tyr Ala Arg Gln Leu
        275                 280                 285

His Ser Leu Glu His Glu Leu Ser Leu Ser Asp Arg Met Asn Lys Val
    290                 295                 300

Thr Pro Gln Leu Leu Ala Leu Ala Asp Ala Gly His Asn Asp Val Pro
305                 310                 315                 320

Ser Arg Val Asp Glu Pro Tyr Arg Arg Ala Val His Gly Val Arg Gly
                325                 330                 335

Arg Ile Leu Ala Thr Thr Ala Glu Leu Ile Gly Glu Asp Ala Val Glu
            340                 345                 350

Gly Val Trp Phe Lys Val Phe Thr Pro Tyr Ala Ser Pro Glu Glu Phe
        355                 360                 365

Leu Asn Asp Ala Leu Thr Ile Asp His Ser Leu Arg Glu Ser Lys Asp
    370                 375                 380

Val Leu Ile Ala Asp Asp Arg Leu Ser Val Leu Ile Ser Ala Ile Glu
385                 390                 395                 400

Ser Phe Gly Phe Asn Leu Tyr Ala Leu Asp Leu Arg Gln Asn Ser Glu
                405                 410                 415

Ser Tyr Glu Asp Val Leu Thr Glu Leu Phe Glu Arg Ala Gln Val Thr
            420                 425                 430

Ala Asn Tyr Arg Glu Leu Ser Glu Ala Glu Lys Leu Glu Val Leu Leu
        435                 440                 445

Lys Glu Leu Arg Ser Pro Arg Pro Leu Ile Pro His Gly Ser Asp Glu
    450                 455                 460

Tyr Ser Glu Val Thr Asp Arg Glu Leu Gly Ile Phe Arg Thr Ala Ser
465                 470                 475                 480

Glu Ala Val Lys Lys Phe Gly Pro Arg Met Val Pro His Cys Ile Ile
                485                 490                 495

Ser Met Ala Ser Ser Val Thr Asp Val Leu Glu Pro Met Val Leu Leu

500                 505                 510

Lys Glu Phe Gly Leu Ile Ala Ala Asn Gly Asp Asn Pro Arg Gly Thr
        515                 520                 525

Val Asp Val Ile Pro Leu Phe Glu Thr Ile Glu Asp Leu Gln Ala Gly
    530                 535                 540

Ala Gly Ile Leu Asp Glu Leu Trp Lys Ile Asp Leu Tyr Arg Asn Tyr
545                 550                 555                 560

Leu Leu Gln Arg Asp Asn Val Gln Glu Val Met Leu Gly Tyr Ser Asp
                565                 570                 575

Ser Asn Lys Asp Gly Gly Tyr Phe Ser Ala Asn Trp Ala Leu Tyr Asp
            580                 585                 590

Ala Glu Leu Gln Leu Val Glu Leu Cys Arg Ser Ala Gly Val Lys Leu
        595                 600                 605

Arg Leu Phe His Gly Arg Gly Gly Thr Val Gly Arg Gly Gly Gly Pro
    610                 615                 620

Ser Tyr Asp Ala Ile Leu Ala Gln Pro Arg Gly Ala Val Gln Gly Ser
625                 630                 635                 640

Val Arg Ile Thr Glu Gln Gly Glu Ile Ile Ser Ala Lys Tyr Gly Asn
                645                 650                 655

Pro Glu Thr Ala Arg Arg Asn Leu Glu Ala Leu Val Ser Ala Thr Leu
            660                 665                 670

Glu Ala Ser Leu Leu Asp Val Ser Glu Leu Thr Asp His Gln Arg Ala
        675                 680                 685

Tyr Asp Ile Met Ser Glu Ile Ser Glu Leu Ser Leu Lys Lys Tyr Ala
    690                 695                 700

Ser Leu Val His Glu Asp Gln Gly Phe Ile Asp Tyr Phe Thr Gln Ser
705                 710                 715                 720

Thr Pro Leu Gln Glu Ile Gly Ser Leu Asn Ile Gly Ser Arg Pro Ser
                725                 730                 735

Ser Arg Lys Gln Thr Ser Ser Val Glu Asp Leu Arg Ala Ile Pro Trp
            740                 745                 750

Val Leu Ser Trp Ser Gln Ser Arg Val Met Leu Pro Gly Trp Phe Gly
        755                 760                 765

Val Gly Thr Ala Leu Glu Gln Trp Ile Gly Glu Gly Glu Gln Ala Thr
    770                 775                 780

Gln Arg Ile Ala Glu Leu Gln Thr Leu Asn Glu Ser Trp Pro Phe Phe
785                 790                 795                 800

Thr Ser Val Leu Asp Asn Met Ala Gln Val Met Ser Lys Ala Glu Leu
                805                 810                 815

Arg Leu Ala Lys Leu Tyr Ala Asp Leu Ile Pro Asp Thr Glu Val Ala
            820                 825                 830

Glu Arg Val Tyr Ser Val Ile Arg Glu Glu Tyr Phe Leu Thr Lys Lys
        835                 840                 845

Met Phe Cys Val Ile Thr Gly Ser Asp Asp Leu Leu Asp Asp Asn Pro
    850                 855                 860

Leu Leu Ala Arg Ser Val Gln Arg Arg Tyr Pro Tyr Leu Leu Pro Leu
865                 870                 875                 880

Asn Val Ile Gln Val Glu Met Met Arg Arg Tyr Arg Lys Gly Asp Gln
                885                 890                 895

Ser Glu Gln Val Ser Arg Asn Ile Gln Leu Thr Met Asn Gly Leu Ser
            900                 905                 910

Thr Ala Leu Arg Asn Ser Gly
        915


<210>  2
<211>  36
<212>  DNA
<213>  Artificial

<220>
<223>  oligonucleotide primer

<400>  2
gcgcgcccat atgactgatt ttttacgcga tgacat                                36


<210>  3
<211>  36
<212>  DNA
<213>  Artificial

<220>
<223>  oligonucleotide primer

<400>  3
tatagtcgac gccggagttg cgcagcgcag tggaaa                                36


<210>  4
<211>  34
<212>  DNA
<213>  Artificial

<220>

<223>  oligonucleotide primer

<400>  4
ggctcaggtg atgtccgggg cagagctgcg tttg                                34


<210>  5
<211>  34
<212>  DNA
<213>  Artificial

<220>
<223>  oligonucleotide primer

<400>  5
caaacgcagc tctgccccgg acatcacctg agcc                                34


<210>  6
<211>  30
<212>  DNA
<213>  Artificial

<220>
<223>  oligonucleotide primer

<400>  6
cacttctcgc acgcggtgtc cagcgccgat                                     30


<210>  7
<211>  30
<212>  DNA
<213>  Artificial

<220>
<223>  oligonucleotide primer

<400>  7
atcggcgctg gacaccgcgt gcgagaagtg                                     30


<210>  8
<211>  27
<212>  DNA
<213>  Artificial

<220>
<223>  oligonucleotide primer

<400>  8
ctctgtccag cgcggatacc cctacct                                        27


<210>  9
<211>  27

<212>  DNA
<213>  Artificial

<220>
<223>  oligonucleotide primer

<400>  9
aggtaggggt atccgcgctg gacagag                                          27


<210>  10
<211>  28
<212>  DNA
<213>  Artificial

<220>
<223>  oligonucleotide primer

<400>  10
cactgcgctg cgcggctccg gctaggtc                                         28


<210>  11
<211>  28
<212>  DNA
<213>  Artificial

<220>
<223>  oligonucleotide primer

<400>  11
gacctagccg gagccgcgca gcgcagtg                                         28


<210>  12
<211>  32
<212>  DNA
<213>  Artificial

<220>
<223>  oligonucleotide primer

<400>  12
aagttcccgg gcaaggggat ttccgataat tc                                    32


<210>  13
<211>  32
<212>  DNA
<213>  Artificial

<220>
<223>  oligonucleotide primer

<400>  13
ggttctctag acaagtacta tgcacgccag ct                                    32

<210>  14
<211>  25
<212>  DNA
<213>  Artificial

<220>
<223>  oligonucleotide primer

<400>  14
ctgttccacg gcggtggtgg caccg                                              25


<210>  15
<211>  25
<212>  DNA
<213>  Artificial

<220>
<223>  oligonucleotide primer

<400>  15
cggtgccacc accgccgtgg aacag                                              25


<210>  16
<211>  21
<212>  DNA
<213>  Artificial

<220>
<223>  oligonucleotide primer

<400>  16
caccgtcggc ggcggtggcg g                                                  21


<210>  17
<211>  21
<212>  DNA
<213>  Artificial

<220>
<223>  oligonucleotide primer

<400>  17
ccgccaccgc cgccgacggt g                                                  21


<210>  18
<211>  34
<212>  DNA
<213>  Artificial

<220>

<223>  oligonucleotide primer

<400>  18
cgagatcatc tccgctgggt acggcaaccc cgaa                                    34

<210>  19
<211>  34
<212>  DNA
<213>  Artificial

<220>
<223>  oligonucleotide primer

<400>  19
ttcggggttg ccgtacccag cggagatgat ctcg                                    34

SEQUENCE LISTING

<110>  Technische Universität Hamburg-Harburg
       TuTech Innovation GmbH

<120>  Modified phosphoenolpyruvate carboxylase enzymes and uses thereof

<130>  P 84374

<160>  19

<170>  PatentIn version 3.3

<210>  1
<211>  919
<212>  PRT
<213>  Corynebacterium glutamicum

<400>  1

Met Thr Asp Phe Leu Arg Asp Asp Ile Arg Phe Leu Gly Gln Ile Leu
1               5                   10                  15

Gly Glu Val Ile Ala Glu Gln Glu Gly Gln Glu Val Tyr Glu Leu Val
            20                  25                  30

Glu Gln Ala Arg Leu Thr Ser Phe Asp Ile Ala Lys Gly Asn Ala Glu
        35                  40                  45

Met Asp Ser Leu Val Gln Val Phe Asp Gly Ile Thr Pro Ala Lys Ala
    50                  55                  60

Thr Pro Ile Ala Arg Ala Phe Ser His Phe Ala Leu Leu Ala Asn Leu
65                  70                  75                  80

Ala Glu Asp Leu Tyr Asp Glu Glu Leu Arg Glu Gln Ala Leu Asp Ala
                85                  90                  95

Gly Asp Thr Pro Pro Asp Ser Thr Leu Asp Ala Thr Trp Leu Lys Leu
            100                 105                 110

Asn Glu Gly Asn Val Gly Ala Glu Ala Val Ala Asp Val Leu Arg Asn
        115                 120                 125

Ala Glu Val Ala Pro Val Leu Thr Ala His Pro Thr Glu Thr Arg Arg
    130                 135                 140

Arg Thr Val Phe Asp Ala Gln Lys Trp Ile Thr Thr His Met Arg Glu
145                 150                 155                 160

Arg His Ala Leu Gln Ser Ala Glu Pro Thr Ala Arg Thr Gln Ser Lys
                165                 170                 175

Leu Asp Glu Ile Glu Lys Asn Ile Arg Arg Arg Ile Thr Ile Leu Trp
            180                 185                 190

Gln Thr Ala Leu Ile Arg Val Ala Arg Pro Arg Ile Glu Asp Glu Ile

195                 200                 205

Glu Val Gly Leu Arg Tyr Tyr Lys Leu Ser Leu Leu Glu Glu Ile Pro
    210                 215                 220

Arg Ile Asn Arg Asp Val Ala Val Glu Leu Arg Glu Arg Phe Gly Glu
225                 230                 235                 240

Gly Val Pro Leu Lys Pro Val Val Lys Pro Gly Ser Trp Ile Gly Gly
                245                 250                 255

Asp His Asp Gly Asn Pro Tyr Val Thr Ala Glu Thr Val Glu Tyr Ser
            260                 265                 270

Thr His Arg Ala Ala Glu Thr Val Leu Lys Tyr Tyr Ala Arg Gln Leu
        275                 280                 285

His Ser Leu Glu His Glu Leu Ser Leu Ser Asp Arg Met Asn Lys Val
    290                 295                 300

Thr Pro Gln Leu Leu Ala Leu Ala Asp Ala Gly His Asn Asp Val Pro
305                 310                 315                 320

Ser Arg Val Asp Glu Pro Tyr Arg Arg Ala Val His Gly Val Arg Gly
                325                 330                 335

Arg Ile Leu Ala Thr Thr Ala Glu Leu Ile Gly Glu Asp Ala Val Glu
            340                 345                 350

Gly Val Trp Phe Lys Val Phe Thr Pro Tyr Ala Ser Pro Glu Glu Phe
        355                 360                 365

Leu Asn Asp Ala Leu Thr Ile Asp His Ser Leu Arg Glu Ser Lys Asp
    370                 375                 380

Val Leu Ile Ala Asp Asp Arg Leu Ser Val Leu Ile Ser Ala Ile Glu
385                 390                 395                 400

Ser Phe Gly Phe Asn Leu Tyr Ala Leu Asp Leu Arg Gln Asn Ser Glu
                405                 410                 415

Ser Tyr Glu Asp Val Leu Thr Glu Leu Phe Glu Arg Ala Gln Val Thr
            420                 425                 430

Ala Asn Tyr Arg Glu Leu Ser Glu Ala Glu Lys Leu Glu Val Leu Leu
        435                 440                 445

Lys Glu Leu Arg Ser Pro Arg Pro Leu Ile Pro His Gly Ser Asp Glu
    450                 455                 460

Tyr Ser Glu Val Thr Asp Arg Glu Leu Gly Ile Phe Arg Thr Ala Ser
465                 470                 475                 480

Glu Ala Val Lys Lys Phe Gly Pro Arg Met Val Pro His Cys Ile Ile
                485                 490                 495

Ser Met Ala Ser Ser Val Thr Asp Val Leu Glu Pro Met Val Leu Leu
            500                 505                 510

Lys Glu Phe Gly Leu Ile Ala Ala Asn Gly Asp Asn Pro Arg Gly Thr
        515                 520                 525

Val Asp Val Ile Pro Leu Phe Glu Thr Ile Glu Asp Leu Gln Ala Gly
    530                 535                 540

Ala Gly Ile Leu Asp Glu Leu Trp Lys Ile Asp Leu Tyr Arg Asn Tyr
545                 550                 555                 560

Leu Leu Gln Arg Asp Asn Val Gln Glu Val Met Leu Gly Tyr Ser Asp
                565                 570                 575

Ser Asn Lys Asp Gly Gly Tyr Phe Ser Ala Asn Trp Ala Leu Tyr Asp
            580                 585                 590

Ala Glu Leu Gln Leu Val Glu Leu Cys Arg Ser Ala Gly Val Lys Leu
        595                 600                 605

Arg Leu Phe His Gly Arg Gly Gly Thr Val Gly Arg Gly Gly Gly Pro
    610                 615                 620

Ser Tyr Asp Ala Ile Leu Ala Gln Pro Arg Gly Ala Val Gln Gly Ser
625                 630                 635                 640

Val Arg Ile Thr Glu Gln Gly Glu Ile Ile Ser Ala Lys Tyr Gly Asn
                645                 650                 655

Pro Glu Thr Ala Arg Arg Asn Leu Glu Ala Leu Val Ser Ala Thr Leu
            660                 665                 670

Glu Ala Ser Leu Leu Asp Val Ser Glu Leu Thr Asp His Gln Arg Ala
        675                 680                 685

Tyr Asp Ile Met Ser Glu Ile Ser Glu Leu Ser Leu Lys Lys Tyr Ala
    690                 695                 700

Ser Leu Val His Glu Asp Gln Gly Phe Ile Asp Tyr Phe Thr Gln Ser
705                 710                 715                 720

Thr Pro Leu Gln Glu Ile Gly Ser Leu Asn Ile Gly Ser Arg Pro Ser
                725                 730                 735

Ser Arg Lys Gln Thr Ser Ser Val Glu Asp Leu Arg Ala Ile Pro Trp
            740                 745                 750

Val Leu Ser Trp Ser Gln Ser Arg Val Met Leu Pro Gly Trp Phe Gly
        755                 760                 765

Val Gly Thr Ala Leu Glu Gln Trp Ile Gly Glu Gly Glu Gln Ala Thr
    770                 775                 780

Gln Arg Ile Ala Glu Leu Gln Thr Leu Asn Glu Ser Trp Pro Phe Phe
785                 790                 795                 800

Thr Ser Val Leu Asp Asn Met Ala Gln Val Met Ser Lys Ala Glu Leu
                805                 810                 815

Arg Leu Ala Lys Leu Tyr Ala Asp Leu Ile Pro Asp Thr Glu Val Ala
            820                 825                 830

Glu Arg Val Tyr Ser Val Ile Arg Glu Glu Tyr Phe Leu Thr Lys Lys
        835                 840                 845

Met Phe Cys Val Ile Thr Gly Ser Asp Asp Leu Leu Asp Asp Asn Pro
    850                 855                 860

Leu Leu Ala Arg Ser Val Gln Arg Arg Tyr Pro Tyr Leu Leu Pro Leu
865                 870                 875                 880

Asn Val Ile Gln Val Glu Met Met Arg Arg Tyr Arg Lys Gly Asp Gln
                885                 890                 895

Ser Glu Gln Val Ser Arg Asn Ile Gln Leu Thr Met Asn Gly Leu Ser
            900                 905                 910

Thr Ala Leu Arg Asn Ser Gly
        915

<210>  2
<211>  36
<212>  DNA
<213>  Artificial

<220>
<223>  oligonucleotide primer

<400>  2
gcgcgcccat atgactgatt ttttacgcga tgacat                                36

<210>  3
<211>  36
<212>  DNA
<213>  Artificial

<220>
<223>  oligonucleotide primer

<400>  3
tatagtcgac gccggagttg cgcagcgcag tggaaa                                36

<210>  4
<211>  34
<212>  DNA
<213>  Artificial

<220>
<223>  oligonucleotide primer

<400>  4
ggctcaggtg atgtccgggg cagagctgcg tttg                                    34


<210>  5
<211>  34
<212>  DNA
<213>  Artificial

<220>
<223>  oligonucleotide primer

<400>  5
caaacgcagc tctgccccgg acatcacctg agcc                                    34


<210>  6
<211>  30
<212>  DNA
<213>  Artificial

<220>
<223>  oligonucleotide primer

<400>  6
cacttctcgc acgcggtgtc cagcgccgat                                         30


<210>  7
<211>  30
<212>  DNA
<213>  Artificial

<220>
<223>  oligonucleotide primer

<400>  7
atcggcgctg gacaccgcgt gcgagaagtg                                         30


<210>  8
<211>  27
<212>  DNA
<213>  Artificial

<220>
<223>  oligonucleotide primer

<400>  8
ctctgtccag cgcggatacc cctacct                                            27


<210>  9
<211>  27
<212>  DNA
<213>  Artificial

<220>
<223>  oligonucleotide primer

<400>  9

aggtaggggt atccgcgctg gacagag                                          27


<210>  10
<211>  28
<212>  DNA
<213>  Artificial

<220>
<223>  oligonucleotide primer

<400>  10
cactgcgctg cgcggctccg gctaggtc                                         28


<210>  11
<211>  28
<212>  DNA
<213>  Artificial

<220>
<223>  oligonucleotide primer

<400>  11
gacctagccg gagccgcgca gcgcagtg                                         28


<210>  12
<211>  32
<212>  DNA
<213>  Artificial

<220>
<223>  oligonucleotide primer

<400>  12
aagttcccgg gcaaggggat ttccgataat tc                                    32


<210>  13
<211>  32
<212>  DNA
<213>  Artificial

<220>
<223>  oligonucleotide primer

<400>  13
ggttctctag acaagtacta tgcacgccag ct                                    32


<210>  14
<211>  25
<212>  DNA
<213>  artificial

<220>
<223>  oligonucleotide primer

<400>  14
ctgttccacg gcggtggtgg caccg                                            25


<210>  15
<211>  25
<212>  DNA
<213>  Artificial

<220>

<223>  oligonucleotide primer

<400>  15
cggtgccacc accgccgtgg aacag                                          25


<210>  16
<211>  21
<212>  DNA
<213>  Artificial

<220>
<223>  oligonucleotide primer

<400>  16
caccgtcggc ggcggtggcg g                                              21


<210>  17
<211>  21
<212>  DNA
<213>  Artificial

<220>
<223>  oligonucleotide primer

<400>  17
ccgccaccgc cgccgacggt g                                              21


<210>  18
<211>  34
<212>  DNA
<213>  Artificial

<220>
<223>  oligonucleotide primer

<400>  18
cgagatcatc tccgctgggt acggcaaccc cgaa                                34


<210>  19
<211>  34
<212>  DNA
<213>  Artificial

<220>
<223>  oligonucleotide primer

<400>  19
ttcggggttg ccgtacccag cggagatgat ctcg                                34

## Description

The present invention relates to methods and enzymes which are useful for producing oxalacetate-derived amino acids such as L-lysine, L-threonine, L-methionine, L-asparagine, L-isoleucine and L-aspartic acid. In particular, the present invention provides a modified phosphoenolpyruvate carboxylase enzyme with a reduced sensitivity to feedback inhibition by aspartic acid, succinic acid and/or malic acid. The invention also provides methods for producing L-lysine, L-threonine, L-methionine, L-asparagine, L-isoleucine or L-aspartic acid which make use of the modified phosphoenolpyruvate carboxylase enzyme. The invention also relates to the use of the modified enzyme for producing one of the above mentioned amino acids.

### BACKGROUND OF THE INVENTION

Amino acids are the basic building blocks for proteins. These nutritional key compounds are widely used for the preparation of feed additives, food ingredients, nutraceuticals and pharmaceuticals. The world production of amino acids has been steadily increasing in recent years and has reached approximately 2 million tons per year. In particular, the amino acids of the aspartic acid group (including L-lysine and L-threonine) have found wide applications, and several hundred thousand tons of these amino acids are produced per year worldwide by microbial fermentation using organisms such as *Corynebacterium glutamicum* and *Escherichia coli.*

Oxaloacetate is one of the most important precursors for the biosynthesis of amino acids of the aspartic acid group, and it must be replenished if the functionality of the citric acid cycle is to be maintained. Many organisms have thus developed anaplerotic pathways that regenerate intermediates for recruitment into the citric acid cycle. Among the important reactions that accomplish replenishing are those in which oxaloacetate is formed from either phosphoenolpyruvate (PEP) by the catalytic action of the enzyme phosphoenolpyruvate carboxylase or from pyruvate which involves the enzyme pyruvate carboxylase. These pathways that resupply intermediates in the citric acid cycle can be utilized both during aerobic and anaerobic metabolism.

Previous research has indicated that the yield and productivity of the oxaloacetate-derived amino acids such as L-lysine or L-threonine depends critically on the carbon flux through anaplerotic pathways (Vallino & Stephanopoulos (1993), Biotechnol. Bioeng. 41:633-646). *C. glutamicum* possesses both phosphoenolpyruvate carboxylase and pyruvate carboxylase for replenishing oxaloacetate that was consumed, e.g. for biosynthesis during growth. Overexpression of both phosphoenolpyruvate carboxylase and pyruvate carboxylase can improve lysine production, wherein the latter showed a more significant improvement. One of the potential reasons is that the activity of phosphoenolpyruvate carboxylase is under the rigid control of cellular intermediates such as aspartic acid, malate and succinate. US Patent 5,919,694 discloses a number of feedback-insensitive mutants of the phosphoenolpyruvate carboxylase enzyme of the *E. coli.* It was demonstrated that overexpression of such mutant enzymes in *C. glutamicum* result in an increase of lysine production of up to 25%, which is much higher than that achieved by overexpressing the wild-type carboxylase.

Despite the above progress in the development of enzymes from *E. coli* which are highly suitable for being used in amino acid production methods, there is still a need for the generation of further enzymes that could be useful for this purpose. In particular, it would be desirable to provide feedback-insensitive enzymes from other microorganisms which are regularly used in fermentation processes for the production of amino acids, such as microorganisms belonging to the genus *Corynebacterium.* Thus, the present invention provides further alternatives for optimizing the production process of amino acids such as L-lysine, L-threonine, L-methionine, L-asparagine, L-isoleucine and L-aspartic acid. By introducing genes coding for the modified enzymes into bacteria producing L-lysine, L-threonine, L-methionine, L-asparagine, L-isoleucine or L-aspartic acid, the fermentative production of these amino acids can be significantly improved.

### DETAILED DESCRIPTION

The present invention provides modified phosphoenolpyruvate carboxylase enzymes which have been derived from the *C. glutamicum* enzyme depicted in SEQ ID NO:1 by one or more amino acid substitutions at pre-determined amino acid positions. Specifically, it was found that an amino acid substitution in any of amino acid positions 813, 869, 873, 917, 614, 620 or 653 of the phosphoenolpyruvate carboxylase enzyme from *C. glutamicum* results in a modified enzyme which is significantly desensitized to feedback inhibition by L-aspartic acid succinic acid, succinic acid and/or malic acid. This means that compared to the wild-type enzyme, higher concentrations of L-aspartic acid, succinic acid and/or malic acid (or their corresponding anions aspartate, succinat and/or malate) are required to inhibit the enzymatical activity of the enzyme. Some of the mutated enzymes provide by the invention may even be completely released from feedback inhibition. Releasing the phosphoenolpyruvate carboxylase enzyme from L-aspartic acid, succinic acid and/or malic acid feedback inhibition, either partially or completely, is a suitable way of increasing the yield of the amino acids of the aspartic acid group, such as L-lysine and L-threonine, in a production process using microorganisms, as the enzyme catalyses a decisive step in the biosynthesis of these amino acids.

The amino acid sequence shown herein as SEQ ID NO:1 is the amino acid sequence of wild-type phosphoenolpyruvate carboxylase of *C. glutamicum.* The present invention has identified positions in the primary structure of this enzyme which can be mutated to achieve a significant or complete release from feedback inhibition effected by L-aspartic acid and other intermediate compounds of the amino acid synthesis of the L-aspartic acid group of amino acids, such as malate and succinate. Therefore, the present invention provides novel enzymatically active polypeptides and enzymatically active fragments thereof which are useful for improving the production of amino acids, in particular L-lysine, L-threonine, L-methionine, L-asparagine, L-isoleucine and L-aspartic acid. Based on the present disclosure, genetically modified microorganisms, in particular strains of *C. glutamicum* or *E. coli* can be generated for use in optimized fermentative production processes.

Host cells which express the phosphoenolpyruvate carboxylase enzyme modified according to the invention will produce one or more of the amino acids L-lysine, L-threonine, L-methionine, L-asparagine, L-isoleucine and L-aspartic acid upon culturing of the cells in a suitable production medium. While L-lysine is normally obtained in high yields, the amount of L-threonine recovered from the culture will be lower. Consequently, additional modifications may be required to increase the yield for L-threonine. For example, it may be required to provide the producing cells with one or more additional copies of the threonine operon, as described in US 7,011,961. In addition, it may also be required to provide for mutated aspartokinase enzymes, such as aspartokinase I and/or aspartokinase III. Modified aspartokinase III enzymes are described in co-pending European patent application 10003770.4. Aspartokinase I enzymes having a reduced L-threonine feedback inhibition sensitivity have been described in US 5,175,107, US 7,186,531 and US 7,220,571.

In a first aspect, the invention thus provides a polypeptide which is an enzymatically active phosphoenolpyruvate carboxylase with a reduced sensitivity to L-aspartic acid, succinic acid and/or malic acid feedback inhibition, said polypeptide comprising the amino acid sequence of SEQ ID NO:1 or an enzymatically active fragment or homolog thereof, wherein one or more amino acids have been replaced at positions that correspond to amino acids 813, 869, 873, 917, 614, 620 or 653 of SEQ ID NO:1. In general, an amino acid occurring at one of the positions which correspond to positions 813, 869, 873, 917, 614, 620 or 653 of SEQ ID NO:1 can be replaced by any other naturally or non-naturally occurring amino acids or by an amino acid analogue, provided that the modified phosphoenolpyruvate carboxylase resulting from the replacement is still enzymatically active and exhibits a reduced sensitivity with respect to the feedback inhibition imposed by L-aspartic acid, succinic acid and/or malic acid.

As the modified phosphoenolpyruvate carboxylase enzymes provided by the present invention are for use in production processes of amino acids, such as L-lysine and L-threonine, it is crucial that the above-mentioned amino acid substitutions, either at positions relevant for L-aspartic acid, succinic acid and/or malic acid feedback inhibition or not, do not result in a significant loss of activity of the enzyme due to a spatial rearrangement of the polypeptide structure. The polypeptides provided by the invention are enzymatically active, which means that they have retained at least part of the enzymatic activity of the phosphoenolpyruvate carboxylase depicted in SEQ ID NO:1 (as measured in the absence of L-aspartic acid, succinic acid and/or malic acid). Preferably, the polypeptides provided by the present invention have retained 50%, 60%, 70%, 80%, 90%, 95%, 96%, 97%, 98%, or 99% of the activity of the phosphoenolpyruvate carboxylase of SEQ ID NO:1, as measured in the absence of L-aspartic acid, succinic acid and/or malic acid. According to a particularly preferred embodiment, the amino acid replacements disclosed by the present invention, which result in a complete or partial release from L-aspartic acid, succinic acid and/or malic acid feedback inhibition, have no influence on the enzymatic activity of the mutated phosphoenolpyruvate carboxylase.

It has been found that the phosphoenolpyruvate carboxylase enzymes of the invention, which have been modified by the replacement of any of the amino acids at positions 813, 869, 873, 917, 614, 620 or 653 of SEQ ID NO:1, show a significant reduction in sensitivity to L-aspartic acid, succinic acid and/or malic acid feedback inhibition. Preferably, the polypeptide enzymes of the invention are at least 2-fold, at least 5-fold, at least 10-fold, at least 15-fold, at least 20-fold, at least 25-fold, at least 50-fold, at least 75-fold, at least 100-fold, at least 150-fold, at least 200-fold, at least 250-fold, at least 500-fold, at least 750-fold, at least 1000-fold, or at least 5000-fold less sensitive to L-aspartic acid, succinic acid and/or malic acid feedback inhibition as compared to the original enzyme shown in SEQ ID NO:1 when measured, for example, in the assay described in the examples below. Even more preferably, the polypeptides of the invention are substantially free from L-aspartic acid, succinic acid and/or malic acid feedback inhibition (i.e. resistant), which means that the presence of L-aspartic acid, succinic acid and/or malic acid in the culture medium does not impair or otherwise negatively influence the enzyme activity of the mutated phosphoenolpyruvate carboxylase enzymes.

The polypeptide of the invention may comprise an amino acid sequence as shown in SEQ ID NO:1, in which one or more of the amino acids located in positions 813, 869, 873, 917, 614, 620 or 653 of SEQ ID NO:1 have been replaced by other amino acids. The amino acids which can be used for replacing the respective amino acids in the naturally occurring enzyme of C. *glutamicum* are not limited to specific amino acids. In principle, any other proteinogenic or non-proteinogenic amino acid may be used for substituting the naturally occurring amino acid in the respective position of the enzyme. Preferably, the amino acids found in the original enzyme of *C. glutamicum* can be replaced by any other naturally occurring, proteinogenic amino acid. As used herein, proteinogenic amino acids are those 23 amino acids which are regularly found in naturally occurring polypeptides, i.e. isoleucine (Ile), alanine (Ala), leucine (Leu), asparagine (Asn), lysine (Lys), aspartic acid (Asp), methionine (Met), cysteine (Cys), phenylalanine (Phe), glutamic acid (Glu), threonine (Thr), glutamine (Gln), tryptophan (Trp), glycine (Gly), valine (Val), proline (Pro), serine (Ser), tyrosine (Tyr), arginine (Arg), histidine (His), selenocysteine, selenomethionine, and pyrrollysine. Preferably, the amino acids are L-amino acids. However, also D-amino acids may be useful for replacing the amino acids in the original polypeptide of SEQ ID NO:1.

Alternatively, the amino acids used for replacing the amino acids in the naturally occurring enzyme of *C. glutamicum* are non-proteinogenic amino acids, i.e. amino acids which are not found in naturally occurring polypeptides. These non-proteinogenic amino acids include, for example, α-aminoadipic acid, β-aminoadipic acid, α-aminobutyric acid, α-aminoisobutyric acid, β-alanine, 4-aminobutyric acid, 5-aminovaleric acid, 6-aminohexanoic acid, 8-aminooctanoic acid, 9-aminononanoic acid, 10-aminodecanoic acid, 12-aminododecanoic acid, α-aminosuberic acid, β-cyclohexylalanine, citrulline, dehydroalanine, α-cyclohexylglycine, propargylglycine, pyroglutamic acid, 4-benzoylphenylalanine, δ-hydroxylysine, 4-hydroxyproline, allo-isoleucine, lanthionine (Lan), norleucine, norvaline, ornithine, phenylglycin, pipecolic acid, sarcosine, 1,2,3,4-tetrahydro-isochinoline-3-carboxylic acid, allo-threonine, thiazolidine-4-carboxylic acid, γ-aminobutyric acid (GABA), iso-cysteine, diaminopropionic acid, 2,4-diaminobutyric acid, 3,4-diaminobutyric acid, biphenylalanine and 4-fluoro-phenylalanine.

Also included by the term "non-proteinogenic amino acids" are derivatives of the above-mentioned proteinogenic amino acids wherein a side-chain has been modified, for example, by a methylene group, thereby providing e.g. homomethionine, homoserine, homoproline, homothreonine, homotryptophane, homotyrosine, homohistidine and homolysine. However, it is preferred according to the invention that an amino acid located in SEQ ID NO:1 at one of the positions 813, 869, 873, 917, 614, 620 or 653 is replaced by one of the other 22 proteinogenic amino acids.

In a particularly preferred aspect of the present invention, the phosphoenolpyruvate carboxylase depicted in SEQ ID NO:1 has been modified by one or more of the following replacements:
(a) Lys at position 813 to Gly, Ala, Val, Leu, Ile, Phe, Trp, Met, Pro;
(b) Arg at position 873 to Gly, Ala, Val, Leu, Ile, Phe, Trp, Met, Pro;
(c) Ser at position 869 to Gly, Ala, Val, Leu, Ile, Phe, Trp, Met, Pro;
(d) Asn at position 917 to Gly, Ala, Val, Leu, Ile, Phe, Trp, Met, Pro;
(e) Arg at position 620 to Gly, Ala, Val, Leu, Ile, Phe, Trp, Met, Pro;
(f) Arg at position 614 to Gly, Ala, Val, Leu, Ile, Phe, Trp, Met, Pro;
(g) Lys at position 653 to Gly, Ala, Val, Leu, Ile, Phe, Trp, Met, Pro.

In a particularly preferred embodiment, the modified phosphoenolpyruvate carboxylase of the present invention comprises one or more of the following amino acid replacements: (a) Lys at position 813 to Gly; (b) Arg at position 873 to Gly; Ser at position 869 to Gly; (d) Asn at position 917 to Gly; (e) Arg at position 614 to Gly; (f) Arg at position 620 to Gly; (g) Lys at position 653 to Gly.

It will be appreciated that the invention is not restricted to the specific phosphoenolpyruvate carboxylase sequence depicted in SEQ ID NO:1. Instead, also enzymatically active homologs of the amino acid sequence of SEQ ID NO:1 may be used, which include (apart from the one or more amino acid replacements in positions 813, 869, 873, 917, 614, 620 or 653) additional sequence differences when compared to the amino acid sequence depicted in SEQ ID NO:1. For example, the invention is suitable for use with phosphoenolpyruvate carboxylases from different species or strains of *Corynebacterium,* which might differ from SEQ ID NO:1 by one or more amino acid substitutions, or with enzymes isolated from a closely related genus.

Homologs of the sequence of SEQ ID NO:1 typically differ from the sequence of SEQ ID NO:1 by one or more deletions, substitutions or additions of amino acids in positions other than those corresponding to positions 813, 869, 873, 917, 614, 620 or 653 of SEQ ID NO:1. Accordingly, apart from the residues which are relevant for the feedback inhibition, one or more further amino acids of the enzyme in SEQ ID NO:1 may be substituted or deleted as long as such modification does not impair the intended release from feedback inhibition, and the homolog resulting from the deletion or substitution is still an enzymatically active phosphoenolpyruvate carboxylase, i.e. the modification should not substantially diminish the enzyme activity of the carboxylase. Generally, any amino acid residue of the amino acid sequences shown in SEQ ID NO:1 can be replaced by a different amino acid, provided that the resultant sequence of the variant is still an enzymatically active polypeptide with phosphoenolpyruvate carboxylase function. In particular, the enzyme depicted in SEQ ID NO:1 may be modified by the substitution of a total of 1, 2, 3, 4, 5, 6, 7, 8, 9, 10, 15, 20, 25, 30, 35, 40, 45 or 50 amino acids, and in some embodiments even in up 50, 60, 70, 80, 90 or 100 amino acids of the enzyme depicted in SEQ ID NO:1. Preferably, these substitutions are not relevant for the release from feedback inhibition by L-aspartic acid, succinic acid and/or malic acid.

If a polypeptide is used in the practice of the invention which includes substitutions at positions other than those disclosed herein in the context with release from feedback inhibition, i.e. in positions other than those corresponding to 813, 869, 873, 917, 614, 620 or 653 of SEQ ID NO:1, it is preferred that such substitutions are conservative substitutions, i.e. substitutions of one or more amino acid residues by an amino acid of a similar polarity, which acts as a functional equivalent. Preferably, the amino acid residue used as a substitute is selected from the same group of amino acids as the amino acid residue to be substituted. For example, a hydrophobic residue can be substituted with another hydrophobic residue, or a polar residue can be substituted with another polar residue having the same charge. Functionally homologous amino acids which may be used for a conservative substitution comprise, for example, non-polar amino acids such as glycine, valine, alanine, isoleucine, leucine, methionine, proline, phenylalanine, and tryptophan. Examples of uncharged polar amino acids comprise serine, threonine, glutamine, asparagine, tyrosine and cysteine. Examples of charged polar (basic) amino acids comprise histidine, arginine and lysine. Examples of charged polar (acidic) amino acids comprise aspartic acid and glutamic acid.

Likewise, polypeptides which differ from the sequence depicted in SEQ ID NO:1 by the insertion of one or more additional amino acids are considered homologs in the context of the present invention. Such insertions can be made at any position of the polypeptide shown in SEQ ID NO:1. Likewise, homologs also include polypeptides in which one or more amino acids have been deleted relative to the polypeptide shown in SEQ ID NO:1. In principle, such deletions can be applied to any amino acid position of the sequence of SEQ ID NO:1.

A homolog of the sequence of SEQ ID NO:1 shows a high degree of sequence identity with the sequence of SEQ ID NO:1. The amino acid identity will be at least about 75%, 80%, 85%, 90%, 91%, 92%, 93%, 94%, 95%, 96%, 97%, 98% or 99% when compared in an optimal alignment, for example, by the program BESTFIT using standard parameters. For example, a sequence identity of 90% means that 90% of the amino acids of an analyzed amino acid sequence stretch are identical to the sequence of the reference amino acid sequence depicted in SEQ ID NO:1. Methods and computer programs for determining amino acid sequence identity are well known in the art.

Also encompassed by the invention are enzymatically active fragments of the phosphoenolpyruvate carboxylase shown in SEQ ID NO:1 as well as enzymatically active fragments of the above-mentioned homologs of the phosphoenolpyruvate carboxylase shown in SEQ ID NO:1, provided that these fragments cover a sequence which includes one or more of the substitutions in positions corresponding to positions 813, 869, 873, 917, 614, 620 or 653 in SEQ ID NO:1. Enzymatically active fragments of the sequence shown in SEQ ID NO:1 or its homologs are polypeptides that differ from the amino acid sequence shown in SEQ ID NO:1 (or from the respective homolog sequence) by the absence of one or more amino acids at the N-terminus and/or the C-terminus of the polypeptide. For example, a fragment of the sequence of SEQ ID NO:1 may differ from the sequence of SEQ ID NO:1 by the lack of about 5, 10, 15, 20, 25, 30, 35, 40, 45 or 50 amino acids at the N-terminus and/or the C-terminus, provided that such fragment retains at least a part of the enzymatic activity of the original full-length enzyme depicted in SEQ ID NO:1. Likewise, a fragment of a homolog of SEQ ID NO:1 may differ from said homolog sequence by the lack of about 5, 10, 15, 20, 25, 30, 35, 40, 45 or 50 amino acids at the N-terminus and/or the C-terminus, provided that the fragment is still enzymatically active.

The present invention also encompasses a polynucleotide encoding a polypeptide, wherein said polypeptide is an enzymatically active phosphoenolpyruvate carboxylase with a reduced sensitivity to L-aspartic acid, succinic acid and/or malic acid feedback inhibition, said polypeptide comprising the amino acid sequence of SEQ ID NO:1 or an enzymatically active fragment or homolog thereof.

The invention further refers to an expression vector, which comprises a polynucleotide coding for a phosphoenolpyruvate carboxylase enzyme of the invention with a reduced sensitivity to L-aspartic acid, succinic acid and/or malic acid feedback inhibition (or an enzymatically active fragment or homolog thereof). Generally, an expression vector refers to a polynucleotide construct, normally comprised of DNA, which allows for the genetic transfer, replication and expression of an inserted polynucleotide in a host cell, wherein said polynucleotide is not native to said host cell, or which is native to said host cell, but has been modified. Replication of the vector can be achieved either by integration of the vector into the genome of the host cell, or by episomal replication (e.g. plasmids). In the latter case, the expression vector comprises a functional origin of replication which allows for its replication in the respective host cell.

The expression vector for use in the present methods is preferably a vector having a high copy number within the host cells so as to provide for high expression levels of the phosphoenolpyruvate carboxylase in the host cell. The expression vector will normally comprise a gene coding for a selectable marker which is useful for identifying and selecting host cells which have been transformed with the vector. Useful markers include those which confer resistance to antibiotics, such as ampicillin, kanamycin, and tetracycline. Such markers are known in the art, and the choice of the proper marker will depend on the host cell and the vector used.

Preferred expression vectors for use in the present invention comprise a polynucleotide of the invention in a form that allows for the expression of said polynucleotide in a host cell. Normally, the expression vector will include regulatory sequences, such as promoters and enhancers, which are operatively linked to the polynucleotide selected for expression. The promoter used in the expression vector may be a constitutive or an inducible promoter. It will be appreciated that the design of the choice of the specific elements for inclusion into the expression vector will depend on the host cell to be transformed, the desired level of polypeptide expression, and the like. Preferably, the expression vectors are designed for the expression of a phosphoenolpyruvate carboxylase enzyme of the invention in prokaryotic cells. More preferably, the expression vectors allow for the expression of the phosphoenolpyruvate carboxylase of the present invention in bacterial cells, such as in cells of *E*. *coli* and *C*. *glutamicum.*

The expression vectors may also provide for the expression of the phosphoenolpyruvate carboxylase in the form of a fusion polypeptide. A fusion polypeptide refers to a non-naturally occurring hybrid polypeptide comprising at least two different polypeptides or polypeptide fragments which do occur naturally associated to each other. Expression vectors which result in the expression of fusion polypeptides normally add several amino acids to the polypeptide to be expressed, either at the N-terminus or at the C-terminus. The additional amino acid may be, for example, helpful for enhancing the expression of the phosphoenolpyruvate carboxylase polypeptide in the host cell, or for purifying said polypeptide after expression.

Also provided are host cells which contain a polynucleotide or an expression vector of the present invention. Different host cells can be used for the production of the phosphoenolpyruvate carboxylase of the invention from the expression vector. In general, both eukaryotic (e.g. yeasts or animal cells) and prokaryotic host cells can be used for recombinantly producing the phosphoenolpyruvate carboxylase. However, it is preferred that the host cell is a prokaryotic cell. Bacterial cells are particularly preferred. For example, the host cells may be bacteria of the genera *Escherichia, Serratia, Brevibacterium* and *Corynebacterium,* as these bacterial hosts are frequently used in established production processes for L-lysine and L-threonine. As shown in the examples, the gene of *Corynebacterium glutamicum* can readily be introduced and expressed in *E. coli*. Host cells of the genus *Escherichia* or Corynebacterium are the most preferred host cells according to the present invention. Strains of *E*. *coli* that can be used for the production of amino acids, such as L-lysine or L-threonine, comprise K-12, JM109, GT3 and the like.

The host cell which is used for the amino acid production process by fermentation may be a cell which naturally possesses all biosynthetic enzymes which are required for producing one or more of the amino acids selected from the group of L-lysine, L-threonine, L-methionine, L-asparagine, L-isoleucine and L-aspartic acid. These cells will be able to synthesize one or more of the respective amino acids. For example, a wild-type *C*. *glutamicum* cell comprising the gene depicted in SEQ ID NO:1 may be used. However, when using the wild-type strain, the yield of the desired amino acid will normally be undesirably low, due to the L-aspartic acid, succinic acid and/or malic acid feedback inhibition of the native phosphoenolpyruvate carboxylase enzyme. By transforming the cells with a polynucleotide coding for a modified phosphoenolpyruvate carboxylase enzyme of the invention, the yield can be significantly increased, since the modified enzyme has a reduced sensitivity to L-aspartic acid, succinic acid and/or malic acid feedback inhibition. Alternatively, the host cell used for the production process may be a cell which does not contain a *ppc* gene or which contains a gene coding for a non-functional phosphoenolpyruvate carboxylase. In these cells, a successful transformation with a polynucleotide of the invention is confirmed by the observation that the cells start to produce the amino acid(s) of interest.

Depending on the host organism selected for transformation, a suitable expression vector will be provided. Examples for suitable vectors for polypeptide expression in *E. coli* cells comprise, for example, the vectors of the pBluescript series, the vectors of the pUC series, e.g. pUC18, pUC19, pBR322, pBR329, pQE70, pQE60, pQE-9, pNH8A, pNH16a, pNH18A, pNH46A, ptrc99a, pKK223-3, pKK233-3, pDR540, pRIT5, pLG338, pKC30, pHSG299, pHSG399, pRep4, pACYC177, pACYC184, pRSF1010, pBW22, and the like. Examples for expression vectors suitable for *C. glutamicum* comprise, for example, the mobilizable *E. coli*/*C. glutamicum* shuttle vectors, e.g. pEC-XT99A, pEC-XC99E, pET-XK99E. Further examples are the vectors described, e.g. in Kirchner et al., 2003, J. Biotechnol., 104:287-299, and in "Cloning Vectors" (Pouwels et al. (eds.) Elsevier, Amsterdam New York Oxford, 1985). The expression vector may be transformed into the host cell by any suitable method. The expression vector of the invention may be introduced into the host cell, e.g. by electroporation, microinjection, particle bombardement or by chemical methods such as calcium phosphate-mediated transformation, as described in Maniatis et al. 1982, Molecular Cloning, A laboratory Manual, Cold Spring Harbor Laboratory.

In a further aspect, the invention relates to a method of producing an amino acid selected from the group consisting of L-lysine, L-threonine, L-methionine, L-asparagine, L-isoleucine and L-aspartic acid, said method comprising the steps of (i) culturing a host cell as defined above, which is capable of producing said amino acid, in a culture medium under conditions which allow for the expression of a polypeptide which is an enzymatically active phosphoenolpyruvate carboxylase with a reduced sensitivity to L-aspartic acid, succinic acid and/or malic acid feedback inhibition, said polypeptide comprising the amino acid sequence of SEQ ID NO:1 or an enzymatically active fragment or homolog thereof, wherein one or more amino acids have been replaced at positions that correspond to amino acids 813, 869, 873, 917, 614, 620 or 653, and (ii) obtaining the amino acid from the culture medium.

Appropriate cell culture media are well known in the art for different kinds of host cells, in particular for bacteria such as *E*. *coli.* For example, strains of *E*. *coli* can be conveniently grown in MB media, LB media and BHI media. A suitable standard minimal media for *E*. *coli* is M9. The specific media for use in culturing a host cell transformed with a polynucleotide encoding the modified phosphoenolpyruvate carboxylase of the invention will depend on the particular strain which is selected for the production process. The skilled person will have no problems to find appropriate media for a host cell based on the literature available in the art.

Culturing of the cells expressing the phosphoenolpyruvate carboxylase of the invention can be performed in accordance with standard fermentation techniques. For example, the cells can be cultured in a batch or fed-batch process. Culturing methods are generally described in Encyclopedia of Bioprocess Technology - Fermentation, Biocatalysis, and Bioseparation, Volumes 1-5, Flickinger, M. C., Drew, S. W. (eds.), 1999 John Wiley & Sons. Typical temperature conditions will be in the range of 20°C to 42°C, more commonly 30°C to 40°C, and preferably 35°C to 38°C. The culture broth will typically have a pH of between 6.5 and 9.0, more typically 7.0 to 8.0, e.g. 7.5. Fermentation of the culture will be continued for a time period ranging from several hours to several days. In particular, if the culturing is performed as a batch process, the culturing time normally ranges from about 12 hours to about 36 hours. When using a continuous process, fermentation times might be up to 21 days or longer.

The purification of the product(s), i.e. L-lysine, L-threonine, L-methionine, L-asparagine, L-isoleucine and/or L-aspartic acid, from the culture media can be achieved by use of commonly known methods, e.g. by use of ion-exchange resins as described in US Patent No. 5,342,766.

Finally, the present invention relates to the use of an phosphoenolpyruvate carboxylase enzyme, which is modified as defined above, an expression vector comprising a polynucleotide encoding such phosphoenolpyruvate carboxylase enzyme and/or a host cell comprising such polynucleotide and/or the expression vector for producing an amino acid selected from the group consisting of L-lysine, L-threonine, L-methionine, L-asparagine, L-isoleucine and L-aspartic acid.

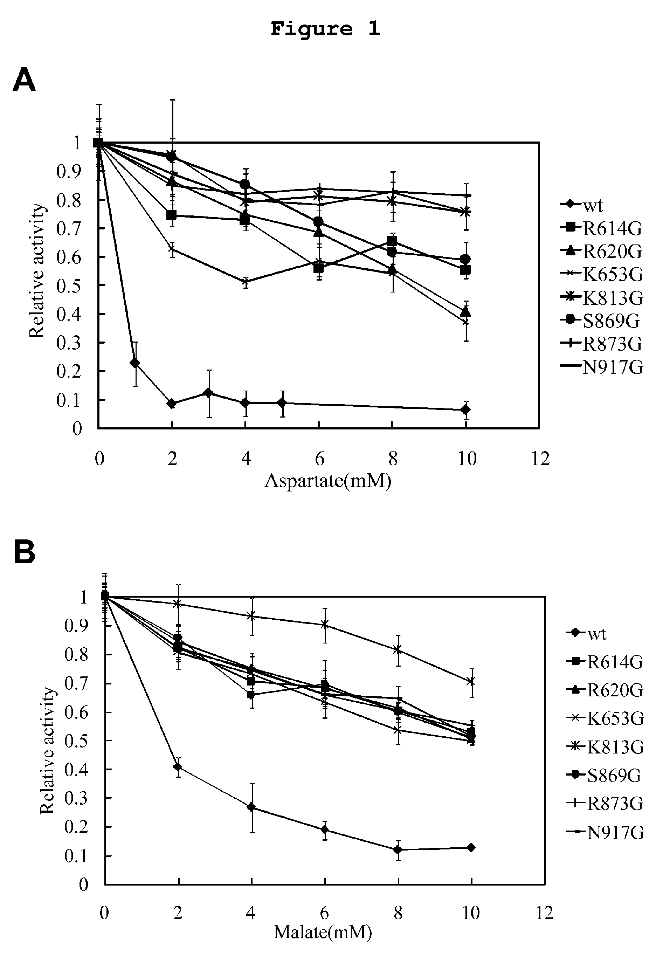

Fig. 1 shows the inhibition profiles of wild-type phosphoenolpyruvate carboxylase and the mutant phosphoenolpyruvate carboxylase enzymes of the invention. The upper panel shows the inhibition profile obtained with L-aspartate, the lower panel that obtained with malate.

### EXAMPLES

### Example 1: Site-directed mutagenesis of the phosphoenolpyruvate carboxylase gene (ppc) of C. glutamicum

The ppc gene encoding the phosphoenolpyruvate carboxylase enzyme was amplified from chromosomal DNA of *C. glutamicum* ATCC 1302 by PCR using the following primers:
5'-GCGCGCCCATATGACTGATTTTTTACGCGATGACAT-3' (SEQ ID NO:2), and
5'-TATAGTCGACGCCGGAGTTGCGCAGCGCAGTGGAAA-3' (SEQ ID NO:3).

The PCR reaction mixture contained:
0.5 µl *C. glutamicum* ATCC 1302 chromosomal DNA (0.1 mg/pl)
0.5 µl primer 1 (SEQ ID NO:2; 10 pm/µl)
0.5 µl primer 2 (SEQ ID NO:3; 10 pm/µl)
0.5 µl pfu polymerase (Fermentas Co, LTD, Germany)
5.0 µl 2 mM dNTP
5.0 µl 10 buffer (10x)
38.0 µl Water

The following PCR program was used for amplification:
95°C 5 min
95°C 1 min
60°C 1 min
72°C 3 min (30 cycles from step 2 to step 4)
72°C 15 min
4°C 10 min

The amplified fragment was purified and digested with the restriction enzymes *Nde*I and *Sal*I. Subsequently, the digested fragments were ligated with the pET-28a+ vector (Invitrogen GmbH, Germany) that had also been digested with *Nde*I and *Sal*I. The recombinant plasmid, designated pET-ppc, was transformed into *E. coli* JM109. The recombinant plasmid was purified and sequenced to confirm the nucleotide sequence.

The plasmid pET-ppc was used as a template for subsequent mutagenesis experiments which introduced site-directed mutations into the amino acid sequence of ppc at a predicted position. The mutagenesis experiments were carried out according to the instruction of QuickChangeII XL site-directed mutagenesis kit (Stratagene).

For the mutation of Lys at amino acid position 813 to Gly, the following primers were used:
5'-GGCTCAGGTGATGTCCGGGGCAGAGCTGCGTTTG-3' (SEQ ID NO:4) and
5'-CAAACGCAGCTCTGCCCCGGACATCACCTGAGCC-3' (SEQ ID NO:5).

The mutant plasmid resulting from the PCR amplification reaction using the primers of SEQ ID NO:4 and 5 was designated pET-ppc (K813G).

For the mutation of Ser at amino acid position 869 to Gly, the following primers were used:
5'-CACTTCTCGCACGCGGTGTCCAGCGCCGAT-3' (SEQ ID NO:6) and
5'-ATCGGCGCTGGACACCGCGTGCGAGAAGTG-3' (SEQ ID NO:7).

The mutant plasmid resulting from the PCR amplification reaction using the primers of SEQ ID NO:6 and 7 was designated pET-ppc(S869G).

For the mutation of Arg to Gly at amino acid position 873, a PCR was conducted by using the following oligonucleotide primers:
5'-CTCTGTCCAGCGCGGATACCCCTACCT-3' and (SEQ ID NO:8) and
5'-AGGTAGGGGTATCCGCGCTGGACAGAG-3' (SEQ ID NO:9)

The plasmid resulting from the PCR amplification reaction using the primers of SEQ ID NO:8 and 9 was pET-ppc(R873G).

For the mutation of Asn to Gly at amino acid position 917, a PCR was conducted by using the following oligonucleotide primers:
5'-CACTGCGCTGCGCGGCTCCGGCTAGGTC-3' (SEQ ID NO:10) and
5'-GACCTAGCCGGAGCCGCGCAGCGCAGTG-3' (SEQ ID NO:11)

The plasmid resulting from the PCR amplification reaction using the primers of SEQ ID NO:10 and 11 was pET-ppc(N917G).

For the mutation of Arg to Gly at amino acid position 614, a PCR was conducted by using the following oligonucleotide primers:
5'-CTGTTCCACGGCGGTGGTGGCACCG-3' (SEQ ID NO:14) and
5'-CGGTGCCACCACCGCCGTGGAACAG-3' (SEQ ID NO:15)

The mutant plasmid resulting from the PCR amplification reaction using the primers of SEQ ID NO:14 and 15 was designated pET-ppc (R614G).

For the mutation of Arg to Gly at amino acid position 620, a PCR was conducted by using the following oligonucleotide primers:
5'-CACCGTCGGCGGCGGTGGCGG-3' (SEQ ID NO:16) and
5'-CCGCCACCGCCGCCGACGGTG-3' (SEQ ID NO:17)

The mutant plasmid resulting from the PCR amplification reaction using the primers of SEQ ID NO:16 and 17 was designated pET-ppc (R620G).

For the mutation of Lys to Gly at amino acid position 653, a PCR was conducted by using the following oligonucleotide primers:
5'-CGAGATCATCTCCGCTGGGTACGGCAACCCCGAA-3' (SEQ ID NO:18) and
5'-TTCGGGGTTGCCGTACCCAGCGGAGATGATCTCG-3' (SEQ ID NO:19)

The mutant plasmid resulting from the PCR amplification reaction using the primers of SEQ ID NO:18 and 19 was designated pET-ppc(K653G).

The sequence of all plasmids encoding a mutated amino acid sequence of the phosphoenolpyruvate carboxylase enzyme of C. *glutamicum* was confirmed by nucleotide sequencing. The plasmids were stored at -80°C or directly used for the subsequent transformation experiments.

### Example 2: Expression of mutated phosphoenolpyruvate carboxylase enzymes

The plasmid pET-ppc and the plasmids encoding the mutated phosphoenolpyruvate carboxylase genes prepared in accordance with Example 1, i.e. pET-ppc(K813G), pET-ppc(S869G), pET-ppc(R873G), pET-ppc(N917G), pET-ppc(R614G), pET-ppc(R620G), pET-ppc(K653G), were transformed into the *E. coli* strain BL21 (DE3) CodonPlus RIPL by heat-shock according to common protocols. The recombinant *E. coli* strains resulting from the transformation experiments were designated:
*E. coli* ppc(K813G),
*E. coli* ppc(S869G),
*E. coli* ppc(R873G),
*E. coli* ppc (N917G),
*E. coli* ppc (R614G),
*E. coli* ppc (R620G), and
*E. coli* ppc (K653G).

The above recombinant *E. coli* strains were cultured at 37°C in LB medium containing 100 mg/L kanamycine until an OD₆₀₀ of 0.6 was reached. Then, IPTG was added to the medium to a final concentration of 0.1 mM, and the bacteria were cultured at 20°C for another 12-14 h. The *E. coli* cells were then harvested and washed with buffer A (20 mM Tris-HCl, pH 7.5) and suspended in buffer B (20 mM Tris-HCl (pH 7.5), 150 mM NaCl, 0.5 M ammonium sulphate). Suspended cells were disrupted by sonication and centrifuged at 13.000 rpm for 1 h. The supernatant was purified by use of a Ni²⁺ NTA column (GE Healthcare Bio-Sciences, Piscataway, NJ). Since phosphoenolpyruvate carboxylase is highly unstable, it is necessary to add additional 0.5 M ammonium sulphate into all of the standard buffers for the stabilization of the enzyme during the purification process.

### Example 3: Characteristics of the purified phosphoenolpyruvate carboxylase enzyme

The activity of phosphoenolpyruvate carboxylase was assayed by a coupling reaction catalyzed by the enzyme malate dehydrogenase. The product (oxaloacetate) is reduced immediately by NADH to malate, and the decrease in NADH is monitored at 340 nm with a spectrophotometer. The standard reaction mixture contained 100 mM Tris-HCl (pH 7.5), 10 mM MnSO₄, 10 mM NaHCO₃, 2 mM phosphoenolpyruvate carboxylase, 0.1 mM NADH, 1.5 IU malate dehydrogenase, appropriate concentrations of aspartate or malate (as inhibitors), and the enzyme. One unit of the enzyme activity was defined as the oxidation of 1 µmol of NADH per min. The specific activity of enzyme is expressed as units/mg of protein.

The inhibition of different phosphoenolpyruvate carboxylase mutants by different aspartate (A) or malate (B) concentrations are shown in Figure 1. It can be seen that the wild-type phosphoenolpyruvate carboxylase enzyme is strictly inhibited by aspartate or malate. In contrast, the mutant enzymes (R614G, R620G, K653G, K813G, S869G, R873G, and N917G) showed a much lower sensitivity towards the inhibitors.

### Example 4: Integration of the ppc mutantion R873G into the chromosome of a Corynebacterium glutamicum strain having an aspartokinase mutation lysC(Q298A)

To construct a plasmid-free mutant, homologous recombination was carried out to integrate the ppc point mutation R873G into the chromosomal DNA of the lysine producing *C. glutamicum* strain lysC (Q298A). The construction of the strain lysC (Q298A) has been described in detail in co-pending European patent application 10003770.4. This strain has a point mutation in the gene encoding the aspartokinase enzyme and is significantly less sensitive towards inhibition by lysine and threonine than wild-type *C. glutamicum.*

A cassette for the homologous recombination was amplified by PCR using genomic DNA of *C. glutamicum* ATCC 13032 as a template and primers:
5'-AAGTTCCCGGGCAAGGGGATTTCCGATAATTC-3' (SEQ ID NO:12)
5'-GGTTCTCTAGACAAGTACTATGCACGCCAGCT-3' (SEQ ID NO:13)

The amplified fragment was purified, digested with the *Sma*I and *Xba*I, purified again and ligated into a pUC18 vector which had been linearized with the same restriction enzymes. The recombinant plasmid was designated pUC18-ppcc. The integrity of the insert was confirmed by sequencing. pUC18-ppcc was used as a template for subsequent mutagenesis experiments which introduced site-directed mutations into the amino acid sequence of ppc at a predicted position. The mutagenesis experiments were carried out according to the instruction of QuickChangeII XL site-directed mutagenesis kit (Stratagene).

The plasmid resulting from the PCR amplification reaction using the primers of SEQ ID NO:8 and 9 was designated pUC18-ppcc(R873G) and its nucleotide sequence was confirmed by sequencing. Plasmid pUC18-ppcc (R873) was digested with restriction enzymes *SmaI* and *XbaI.* The ppc (R873) fragment obtained was subcloned into pK18mobsacB vector (Kirchner et al. (2003), J. Biotechnol. 104: 287-299) previously digested with SmaI-*Xba*I, transformed into the competent cells of *E. coli DH5α-mcr* (Grant et al.(1990), Proc. Natl. Acad. Sci. USA. 87:4645-4649.). The resulting recombinant plasmid was purified and designed as pK18mobsacB-ppc(R873G). The recombinant plasmid pK18mobsacB-ppc(R873G) was subsequently introduced into the lysine producing strain *C. glutamicum* (lysCQ298A) by electroporation (Van der Rest et al. (1999), Appl. Microbiol. Biotechnol. 52, 541-545). The vector cannot replicate independently in this strain and is only retained in the cell when integrated in the chromosome as the result of a recombination event.

The selection of clones in which the plasmid has successfully integrated into the genome by a first homologous recombination event were selected by growth on LBHIS medium containing 15 mg/l of kanamycin, and incubation at 30°C for 48 hours.

Incubation of a positive clone in a kanamycine-free BHIS medium allows a second recombination event. Clones in which the vector backbone has been successfully eliminated by a second recombination event are identified by growth on sucrose-containing medium. Only clones which have lost the vector backbone comprising the *SacB* will survive; the *SacB* gene can be used as a positive selection marker as cells which carry this gene cannot grow on sucrose-containing medium. Depending on the location of the second recombination event in relation to the mutation site, allele replacement or incorporation of the mutation takes place in the excision or the original copy remains in the chromosome of the host.

Clones in which the two recombination events have led to successful replacement of the native ppc-coding region were identified by sequencing of a PCR-product spanning the relevant region. The PCR product was generated using genomic DNA of individual clones as a template and the primers depicted in SEQ ID NOs:12 and 13. The PCR-product was purified and sequenced. PCR products with positive integration were stored. The mutant strain is referred to as *C. glutamicum* lysC(Q298A)ppc(R873G).

### Example 5: Production of lysine by the mutant Corynebacterium glutamicum lysC(Q298A)ppc(R873G)

The wildtype of *C. glutamicum* ATCC 13032 as well as the recombinant strains *C. glutamicum* lysC(Q298A) and *C. glutamicum* lysC(Q298A)ppc(R873G) were cultured to test the production of L-Lysine. The fermentation medium was composed of CSL 5 g/L, MOPO 20 g/L, 50 g/L glucose, 25 g/L (NH₄)₂SO₄, 0.1 g/L KH₂PO₄, 1 g/L MgSO₄•7H₂O, 0.01 g/L FeSO₄•7H₂O, 0.005 g/L MnSO₄•5H₂O, 0.01 g/L CaCl₂•2H₂O, 0.3 mg/L Biotin, 0.3 mg/L Thiamine•HCl, 0.4 g/L L-homoserine, 25 g/L CaCO₃ and 100 mg/L ampicillin. The wildtype and recombinant *C. glutamicum* strains were cultured at 33°C for 48 hours.

The production of L-lysine by the different *C. glutamicum* strains are summarized in Table 1. It is shown that the introduction of a feedback inhibition released phosphoenolpyruvate carboxylase greatly improved the lysine production.

**Table 1: Production of lysine in C. glutamicum**

| **Bacterial strain** | **L-lysine production (g/L)** |
|---|---|
| *C. glutamicum* ATCC 13032 | 0 |
| *C. glutamicum* lysC(Q298A) | 10.3 |
| *C. glutamicum* lysC(Q298A)ppc(R873G) | 14.4 |

## Claims

1. Polypeptide which is an enzymatically active phosphoenolpyruvate carboxylase enzyme with a reduced sensitivity to feedback inhibition by aspartic acid, succinic acid and/or malic acid, said polypeptide comprising the amino acid sequence of SEQ ID NO:1 or an enzymatically active fragment or homolog thereof, wherein one or more amino acids have been replaced at positions that correspond to amino acids 813, 869, 873, 917, 614, 620 or 653 of SEQ ID NO:1.

2. Polypeptide of claim 1, wherein the amino acid replacements are one or more of the following replacements:
(a) Lys 813 to Gly, Ala, Val, Leu, Ile, Phe, Trp, Met, Pro;
(b) Ser 869 to Gly, Ala, Val, Leu, Ile, Phe, Trp, Met, Pro;
(c) Arg 873 to Gly, Ala, Val, Leu, Ile, Phe, Trp, Met, Pro;
(d) Asn 917 to Gly, Ala, Val, Leu, Ile, Phe, Trp, Met, Pro.
(e) Arg at position 620 to Gly, Ala, Val, Leu, Ile, Phe, Trp, Met, Pro;
(f) Arg at position 614 to Gly, Ala, Val, Leu, Ile, Phe, Trp, Met, Pro;
(g) Lys at position 653 to Gly, Ala, Val, Leu, Ile, Phe, Trp, Met, Pro.

3. Polypeptide of claim 1 or 2, wherein the amino acid replacements are one or more of the following replacements:
(a) Lys 813 to Gly;
(b) Arg 873 to Gly;
(c) Asn 917 to Gly;
(d) Ser 869 to Gly;
(e) Arg 620 to Gly;
(f) Arg 614 to Gly;
(g) Lys 653 to Gly.

4. Polypeptide of any of claims 1-3, wherein the sensitivity to feedback inhibition by aspartic acid, succinic acid and/or malic acid is reduced at least 2-fold.

5. Polypeptide of claim 4, wherein the sensitivity to feedback inhibition by aspartic acid, succinic acid and/or malic acid is reduced at least 5-fold.

6. Polynucleotide encoding a polypeptide of claims 1-5.

7. Expression vector comprising the polynucleotide of claim 6.

8. Host cell comprising the polynucleotide of claim 6 or the expression vector of claim 7.

9. Host cell of claim 8, wherein the host cell is a prokaryotic cell.

10. Host cell of claim 9, wherein the host cell is a bacterial cell.

11. Host cell of claim 10, wherein the bacterial cell is an *Escherichia* cell.

12. Host cell of claim 10, wherein the bacterial cell is a *Corynebacterium* cell.

13. Method of producing an amino acid selected from the group consisting of L-lysine, L-threonine, L-methionine, L-asparagine, L-isoleucine and L-aspartic acid, said method comprising the steps of (i) culturing a host cell of any of claims 8-12, which is capable of producing said amino acid, in a culture medium under conditions which allow for the expression of a polypeptide of any of claims 1 to 5, and (ii) obtaining the amino acid from the culture medium.

14. Use of a polypeptide of any of claims 1 to 5, an expression vector of claim 7 or a host cell of claims 8-12, for producing an amino acid selected from the group consisting of L-lysine, L-threonine, L-methionine, L-asparagine, L-isoleucine and L-aspartic acid.
